# EUROPEAN PATENT APPLICATION

(11) **EP 4 737 896 A1**
(43) Date of publication of application: **06.05.2026**
(21) Application number: 24210455.2
(22) Date of filing: 04.11.2024
(51) Int. Cl.: G01N 33/53, C12Q 1/6841

(54) **METHOD FOR SPATIAL ANALYSIS OF GENETICALLY-MODIFIED CELL THERAPY PRODUCTS IN SITU**

(71) Applicant: Miltenyi Biotec B.V. & Co. KG, 51429 Bergisch Gladbach (DE)
(72) Inventor: SCHNEIDER, Dina, 51429 Bergisch Gladbach (DE); LEE, Jia-Jye, 51429 Bergisch Gladbach (DE); HU, Peirong, 51429 Bergisch Gladbach (DE); PARK, Dongju, 51429 Bergisch Gladbach (DE); NEIL, Emily, 51429 Bergisch Gladbach (DE); PINARD, Robert, 51429 Bergisch Gladbach (DE)
(74) Representative: Kisters, Michael Marcus

(57) **Abstract**

The invention is directed to a method to detect the phenotype and the spatial location of cells provided with a chimeric antigen receptor (CAR) in a fixed biological sample by targeting the mRNA coding for the chimeric antigen receptor (CAR) comprising the steps
a. providing a plurality of oligonucleotides, each having at least one detection section and at least one amplification section and two target binding sections at the respective 5' and 3' ends
b. hybridizing the oligonucleotides with their 3' and 5' ends to the mRNA
c. ligating the 3' and 5' ends of the hybridized oligonucleotides to generate circularized molecules
d. hybridizing an amplification oligonucleotide to at least one amplification section of the circularized molecules thus enabling rolling circle amplification (RCA) of the circularized molecules
e. multiplying the circularized molecules by RCA in the fixed biological sample thereby creating DNA nanoballs comprising concatemers of the DNA nanoballs
f. hybridizing detector oligonucleotides comprising at least one fluorescent unit to at least one detection section of the DNA nanoballs, thereby obtaining the spatial location of the cells provided with the chimeric antigen receptor (CAR) in the fixed biological sample
g. providing a plurality of fluorescently-labeled antibodies capable of binding to different phenotype defining antigens, thereby obtaining the phenotypes of the cells in the fixed biological sample
h. aligning the spatial location of cells provided with the chimeric antigen receptor (CAR) and the phenotypes of cells in the fixed biological sample.

## Description

### BACKGROUND

The invention is directed to detecting the phenotype and the spatial location of CAR cells on a fixed tissue, thus enabling a quality control for Chimeric antigen receptor (CAR) T cells intended for the treatment of a patient.

Chimeric antigen receptor (CAR) T cells are engineered cells used in cancer therapy and are studied to treat infectious diseases. Trafficking and persistence of CAR T cells is an important requirement for efficacy to target cancer and in situ, CAR T cell exhibited a heterogenous effector gene expression which is related to the distance from tumor cells.

CAR T cells are a novel therapeutic modality for the treatment of cancer, autoimmune disease, infectious diseases, and more. CAR T cell molecules are typically comprised of an antigen targeting domain, hinge and transmembrane domain and an intracellular domain which mediates T cell activation and effector function, leading to production of soluble factors, and tumor cell killing. CAR T cells may be generated as an autologous (from patient) or allogeneic (from donor) product. The production of CAR T cells involves introduction of transgenic sequences into the genome of the T cells. Expression of these CAR sequences leads to the appearance on the T cell surface of artificial chimeric antigen receptors which redirect CAR T cells toward recognition of tumor cell antigens and killing of tumor cells. In the process of tumor cell recognition and killing, T cells interact with other immune cells, immune-associated tumor stroma, and tumor cells themselves, all of which has impact on therapy outcomes. [Sterner, R.C & Sterner, R.M. Blood Cancer J. 11, 69 (2021).

Spatial biology is a valuable tool for understanding the mechanisms of tumor progression and CAR T cell therapeutic anti-tumor function, with potential applications in basic research, translational research, diagnostics and clinical applications. The tumor microenvironment (TME) can shape the molecular characteristics of T cells , which can include: increasing the expression of co-inhibitory receptors, loss of effector function, poor proliferation and dysregulated metabolic activity. Immunotherapies that block immune checkpoints have been successful in treating advanced-stage tumors. However, the TME can still dampen the efficacy of CAR T cells. Studying both the TME composition using proteins markers as well as specifically targeting the CAR constructs is a very effective way to improve T cell function in the TME, including designing CAR-T cells that can function better in various conditions such as hypoxia for example.

### OBJECT OF THE INVENTION

It was therefore an object of the invention to provide a method to detect and quantify the spatial distribution of the CAR cell population in situ at a single cell resolution for preclinical and clinical applications. These may include, among others, pre-clinical animal models of CAR T cell function, and interrogation of human clinical specimen in diagnostic applications and correlative clinical studies pre-and post-CAR administration.

Accordingly, the invention is directed to a method to detect the phenotype and the spatial location of cells provided with a chimeric antigen receptor (CAR) in a fixed biological sample by targeting the mRNA coding for the chimeric antigen receptor (CAR) comprising the steps
a. providing a plurality of oligonucleotides, each having at least one detection section and at least one amplification section and two target binding sections at the respective 5' and 3' ends
b. hybridizing the oligonucleotides with their 3' and 5' ends to the mRNA
c. ligating the 3' and 5' ends of the hybridized oligonucleotides to generate circularized molecules
d. hybridizing an amplification oligonucleotide to at least one amplification section of the circularized molecules thus enabling rolling circle amplification (RCA) of the circularized molecules
e. multiplying the circularized molecules by RCA in the fixed biological sample thereby creating DNA nanoballs comprising concatemers of the DNA nanoballs
f. hybridizing detector oligonucleotides comprising at least one fluorescent unit to at least one detection section of the DNA nanoballs, thereby obtaining the spatial location of the cells provided with the chimeric antigen receptor (CAR) in the fixed biological sample
g. providing a plurality of fluorescently-labeled antibodies capable of binding to different phenotype defining antigens, thereby obtaining the phenotypes of the cells in the fixed biological sample
h. aligning the spatial location of cells provided with the chimeric antigen receptor (CAR) and the phenotypes of cells in the fixed biological sample.

The biological sample may be fixed, i.e. immobilized on any kind of surface by any methods known in the art. Preferable, the surface is a transparent glass sheet like a microscope slide or a disposable for RNASky system, available from Miltenyi Biotec B.V. & Co. KG.

The term "cells in the fixed biological sample" refers not only to cells embedded in the biological sample, but also cells on the surface of the biological sample.

The detection section, the at least one amplification section and the two target binding sections may comprise between 2 and 100 nucleotides.

Since the sequence of the mRNA coding for the chimeric antigen receptor (CAR) is in general known and the sequence of the target binding sections needs to be complementary for hybridization, the sequence of the target binding sections are also known an can be easily generated.

The method allows for the comparison of genetically modified therapeutic cells, such as CAR T cells, to non-modified T Cells.

### SUMMARY

The invention is a method that utilizes a set of RNA padlock probes targeting CAR constructs to obtain the spatial location of targeted CAR mRNA sequence of specific T cell, and combining it to a specific set of protein markers on the same tissue sample to determine cell phenotyping. The designed padlock probes target multiple region of the CAR construct unique open reading frame rather than the viral untranslated regions (UTRs) present in all CAR used in cell and gene therapy and TCR mRNAs. This open reading frame and is not naturally found in human or viral genomes. Additionally, by combining specific protein markers on the same tissue section to detect and quantify the expression level of the CAR in T cells, this approach allows for a superior specificity and resolution of detection than the known technology.

### BRIEF DESCRIPTION OF THE DRAWINGS

Figure 1 describes the padlock probe design. Sixteen RNASky padlock probes with specific binding sites targeting 11 region of the chimeric receptor were designed. The padlock probes contain two anchor regions that recognize and hybridized to the sequence of interest
Figure 2 shows the CAR construct and the unique regions targeted by the padlock probes design
Figure 3 shows the seven main workflow steps of the described method
Figure 4 shows the expression levels of the transduced CAR T Cells and the untransduced cells (UTD cells) by Flow cytometry.

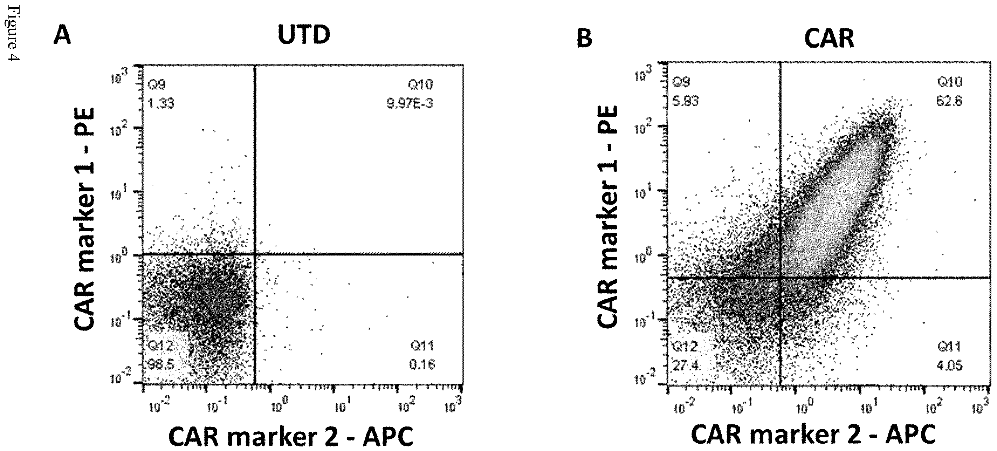

Fig. 4 Flow cytometry confimed (A) no CAR expression of UTD cells and 62.6% of CAR T cells with co- expression of CD19 and CD22

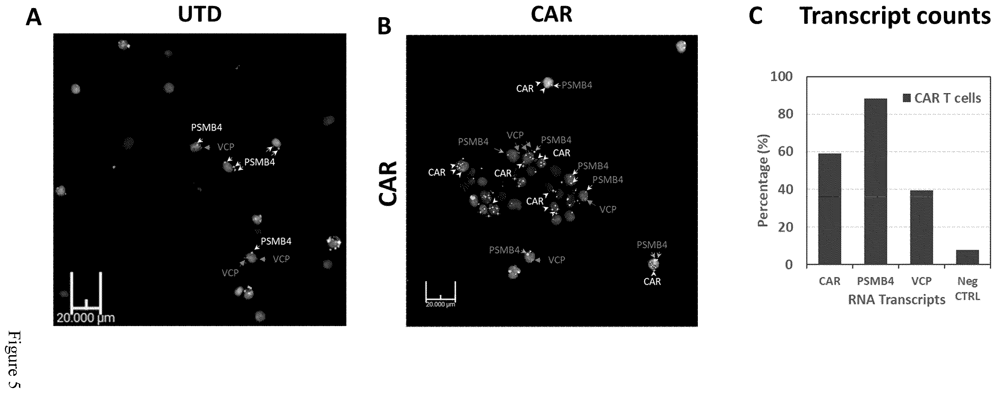

Figure 5 shows the visualization (A&B) and quantification (C) of CAR RNA transcripts targeted by the specific padlock probes

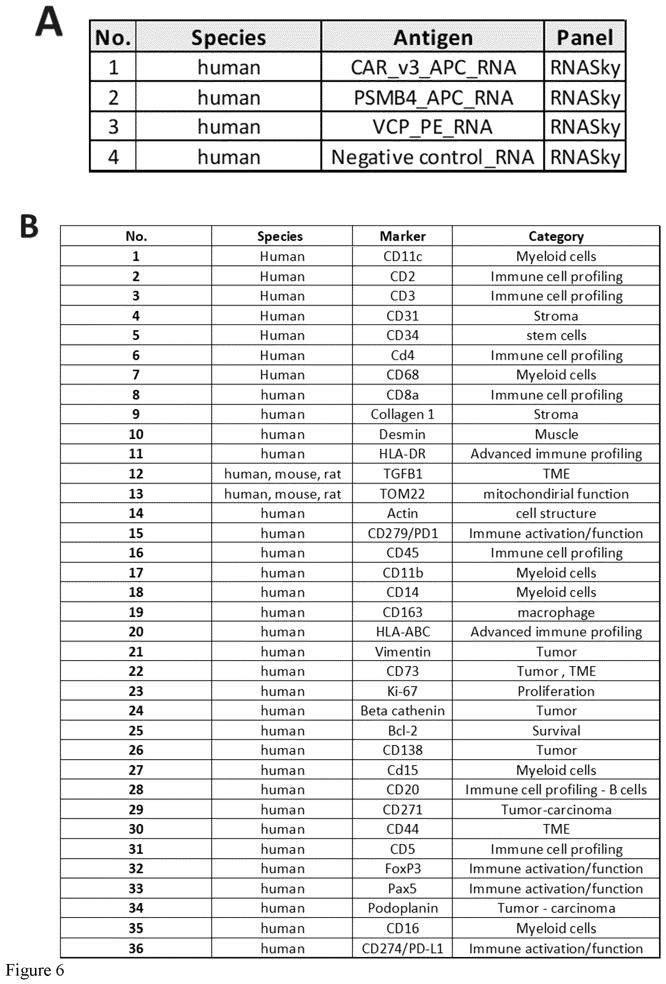

Figure 6 indicates the composition of the four-plex RNA transcript detection of RNA panel (A) and the 36-plex protein detection using fluorescently-labeled antibody panel (B).

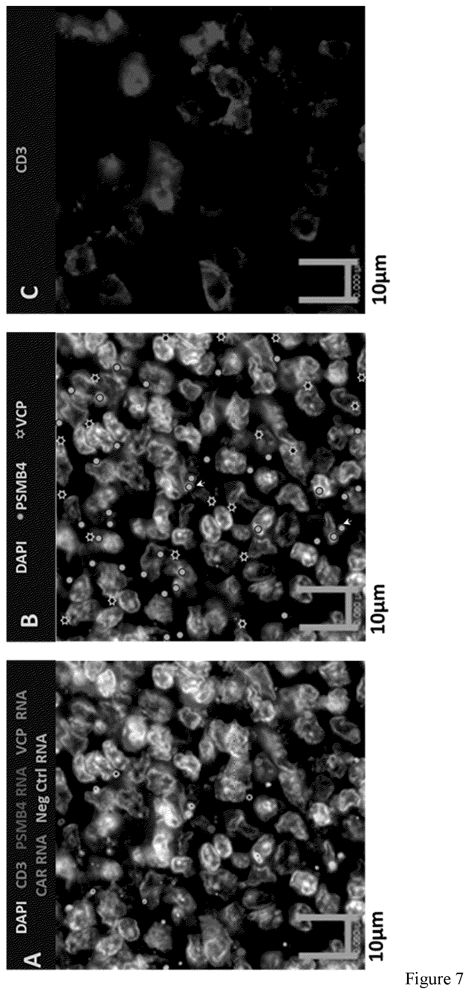

Figure 7 shows the 4-plex RNA transcription and the 36-plex panel protein detection on the same human tonsil FFPE tissue section.

### DETAILED DESCRIPTION

The method described here allows the spatial detection of specific CAR engineered T Cell by targeting multiple regions of the open reading frame of the CAR mRNA, in combination with a specific panel of protein antibody markers, on the same tissue section. First, A panel of targeted padlock probes are used to generate circular template that are then amplified by RCA (rolling circle amplification) into multiple concatemers that form DNA nanoballs. The DNA nanoballs are detected are labeled using fluorescently labeled short oligonucleotides complementary to the back bone of the padlock probes. The spatial localization of the padlocks indicate the presence of mRNA belonging to the CAR specific construct and is used to assess the effectiveness of CAR T cell therapy by quantifying the number of CAR T cells present within a tumor and their proximity to tumor cells. It can also be used to assess areas where CAR T cells might not be reaching effectively, leading to improvements in CAR design to enhance tumor penetration. Additionally using a specific set of fluorescently labeled antibody key proteins are also labeled allowing for the assessment of the actual composition of the tumor microenvironment (TME) and the phenotyping of the various cells present, including immune cells.

Genetic modification for cells for therapy often involves introduction of new genetic material, which stably integrates into the host cell DNA becomes a part of host genome, and can be re-expressed over long periods of time. The expression of protein products of such modification is accomplished by transcription of the DNA open reading frame of the transgene to mRNA, and then translation of the mRNA template to a protein. After additional processing and trafficking within the cell, such protein reaches its intended location within the cell and performs an intended therapeutic function. Because generation of mRNA is a required intermediate step for protein expression, detection of specific mRNA sequences may serve as a highly discriminating probe for the detection of transgenic proteins of interest.

In a first embodiment, the mRNA coding for the chimeric antigen receptor (CAR) comprises one or more sections coding for the leader peptide, activator, hinge, skip, transmembrane, and intracellular signaling domain regions of the CAR and the oligonucleotides are provided with target binding sections at its 5' and 3' ends each capable of hybridizing to at least one of the CAR mRNA sections.

Preferable, the plurality of fluorescently-labeled antibodies capable of binding to phenotype defining antigens are selected from the group consisting of CD11c, CD2, CD3, CD31, CD34, CD4, CD68, CD8a, Collagen1, Desmin, HLA-DR,TGF beta1, TOM22, ACTIN, CD279, CD45, CD279, CD45, CD11b, CD14, CD163, HLA-ABC, Vimentin, CD73, Ki-67, B-Catenin, Bd-2, CD-138, CD15, CD20, CD271, CD44, CD5, Fox P3, PAX-5, Podoplanin, CD16, CD274.

In a further embodiment, the expression level of the chimeric antigen receptor (CAR) within the cells in the fixed biological sample having a certain phenotype is quantified by detecting the amount of detector oligonucleotides bound to the detection sections of the DNA nanoballs.

Preferable, the cells provided with a chimeric antigen receptor (CAR) have the phenotype of a T-cell.

For example, CAR T cells were generated from human CD4+ and CD8+ T cells extracted from the blood of a healthy donor. T cells were transduced with lentiviral vector to stably express CAR molecules on their surface. T cells from the same donor but without transduction served as a negative control for CAR expression (untransduced, UTD). CAR T cells and the UTD control were then subjected to RNA detection analysis using CAR-specific padlock probes along with VCP and PSMB4 padlock probes specific for human cell housekeeping genes as positive controls and one negative control probe. Cells were counter-stained with DAPI to identify cell nuclei. Samples were acquired on a high-content imaging device (MACSima) and analyzed using an image analysis software (MACSIQview). The number of CAR-positive and CAR-negative T cells within the sample was therefore quantified with high precision.

CAR detection uses amplified mRNA detection probes targeting the synthetic construct along with a set of specific fluorescent antibody targeting key protein markers on the same tissue, allowing for a better detection of chimeric mRNA than known technologies. For the RNA detection, padlock probes are hybridized to specific portions of the ORF (open reading frame ) of the CART mRNA and ligated creating a circular molecule. The circularized DNA is replicated by rolling circle amplification directly on fixed tissue to selectively generate a series of concatemers that form DNA nanoballs or rolonies. The rolony concatemers are then labelled with fluorescently labelled detector probes binding to a specific and internal barcode sequence of the padlock probes. The visualization of the labelled probes hybridized to the rolonies by fluorescent microscopy imaging allows for physical localization of the padlock oligonucleotide on the tissue and for the determination the actual identity of the actual padlock bound to the RNA transcript. A series of specific and pre-defined panel of labeled monoclonal antibodies targeting specific protein markers compatible with RNA detection are combined on the same tissue section and used for immune cell segmentation.

The quantitative (expression level) and spatial assessment could serve as an important tool for preclinical assessment of CAR T cell effectiveness. The use of multiple probes targeting specifically the CAR unique sequence and proteins detection on the same tissue, can be used to understand the contextual function of the CAR T cell population within the tumor microenvironment (TME), patient response, as well as persistence which correlated with long-term disease-free survival and durable remissions.

In another embodiment, human T cells from a cancer patient were genetically modified to express chimeric antigen receptors (CAR T cells). A cancer patient was treated with CAR T cells; and tumor specimen was collected post-CAR T cell therapy. MACSima spatial analysis utilized fluorescently labeled monoclonal antibodies recognizing protein epitopes of interest to identify tumor, stroma and infiltrating immune cells. Padlock probes specific for CAR sequence was applied to determine whether the tumor infiltrating T cells were CAR - positive. Furthermore, additional probes were also used to determine localization of specific mRNA transcripts representing tumor, stroma or immune cells. High-content imaging data analysis was used to determine the spatial organization of tissue and the relationship between various cell types, including CAR+ T cells.

In another embodiment, an antibody panel was designed to specifically investigate key aspects of tumor such as the interplay between tumor proliferation and spread, the immune system action to contain and eliminate the tumor, and tumor-proximal stroma, which may be both tumor-supporting and tumor-restricting. The protein panel was used in combination with CAR-specific RNA probes for concurrent identification of RNAs and proteins of interest in the same tissue sections in situ. The combination of the two mode of detection allows for the phenotyping and activation state of immune, stroma and tumors cells

The method of the invention is now described in detail by referring to the figures, and the term "steps" refers to the respective steps of the method.

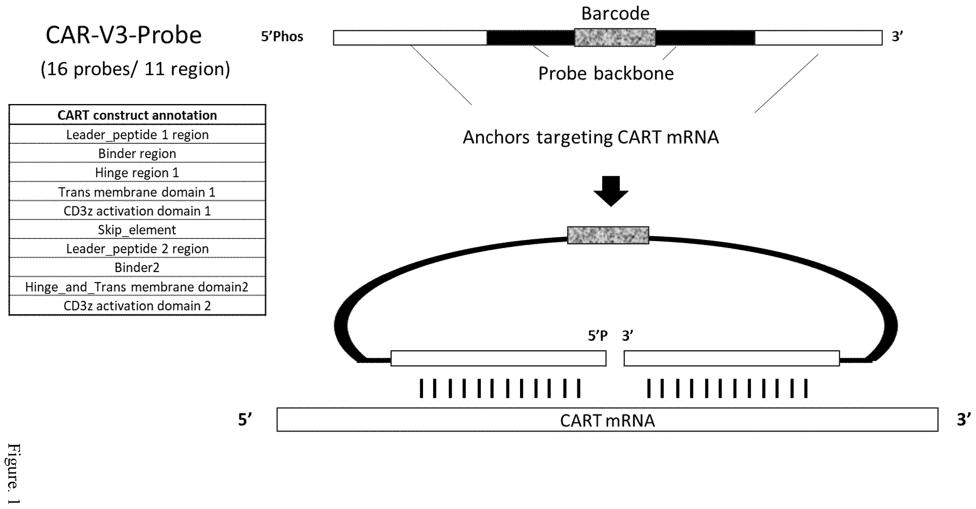

Figure 1: Padlock probe description. Padlock probes are synthetic oligonucleotides that contain two anchor regions that recognize and hybridized to the sequence of interest, and a backbone region in black that contains a pre-defined barcode sequence. Sixteen padlock probes were designed to target specific regions of the CAR construct.

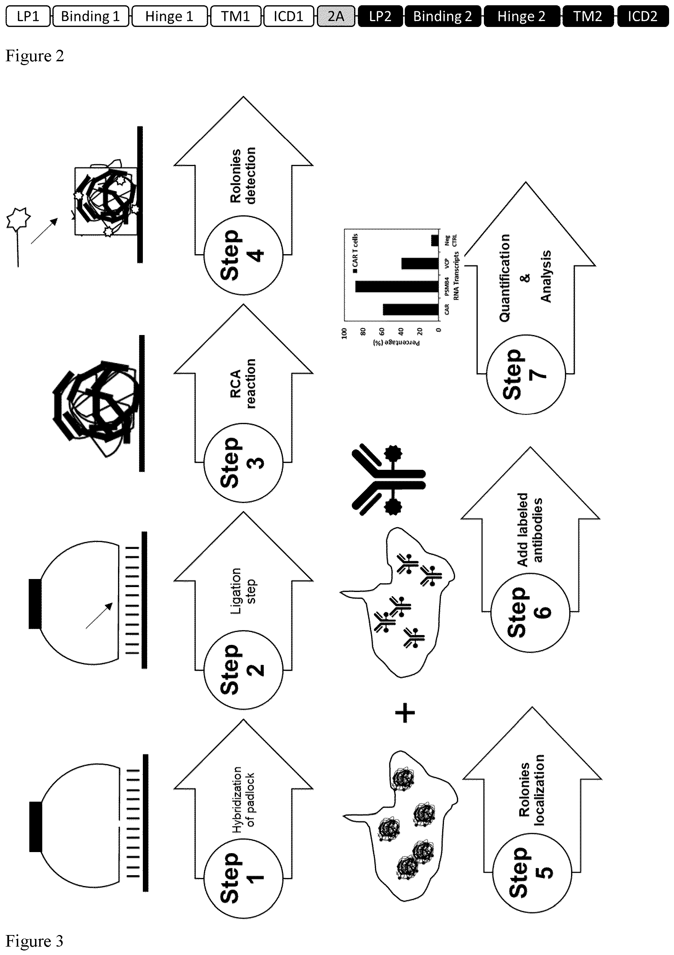

Figure 2: Chimeric antigen receptor (CAR description). The chimeric antigen receptor coding sequence was comprised of two co-encoded CAR molecules, separated by the ribosomal skip element 2A, to facilitate equal expression of the two CARs. Each CAR is comprised of a leader peptide (LP), target binding region, hinge region, transmembrane region, and intracellular signaling domain region (ICD).

Figure 3: Tissue or cell lines or other biological samples are mounted on a glass slide are permeabilized and antigen retrieval is performed using heat to remove cross-links between adjacent proteins removing the steric bonding interference between the antibody and its linear epitope. Step 1: once the sample is ready the padlock probes are added to the sample and incubated for a fix amount of time to allow for hybridization to its given target. Step 2: the two extremities of the padlock, brought in close proximity by hybridization, are ligated using an RNA/DNA hybrid ligase to form a circular template. Step 3: the circular template is amplified by Rolling Circle Amplification (RCA) in presence of dNTP and Amino-allyl dUTP to form DNA nanoballs (rolonies). The DNA nanoballs are crosslinked to the biological sample via the Amino-allyl groups. Step 4: Fluorescently-labeled short oligonucleotides containing a sequence complementary to the barcode present in the padlock backbone are added to the sample. Step 5: the fluorescently labeled short oligonucleotides hybridize to the rolonies and each of the rolonies can now be visualized and their x,y sample coordinates assigned. Step 6: Fluorescently-labeled monoclonal antibodies are added to the sample and the immuno-labeled targeted proteins are visualized. Step 7: The rolonies are assessment of the immune- labeling protein signal is assessed and further analysis determining the gene expression profile and cell phenotyping is performed.

Figure 4: The expression levels of the tested sample (T cell that has been genetically modified to express chimeric antigen receptors (transduced CAR T Cells) and the untransduced cells (UTD cells), used as negative control, were first confirmed by Flow cytometry. A four-color flow cytometry panel was designed using a commercial CD19 CAR Detection Reagent (Miltenyi Biotec BV&Co KG, Bergisch Gladbach, Germany). The reagent consists of a biotinylated CD 19 antigen that specifically binds CD19-targeted CARs. In a second incubation step the biotin-labeled CAR T cells are then stained with a fluorochrome-conjugated anti-biotin antibody. Two hundred µl of whole blood were treated for 10 min with 2 ml of NH4Cl-based erythrocyte lysing solution and washed with PBS, containing 0.5% HSA. After removal of the supernatant down to 200 µl, cells were resuspended and 100 µl were transferred to a a flow cytometry tube. Following 15 min of incubation with CAR Detection reagents. In (A) no CAR expression of UTD cells and (B) 62.6% shows CAR T cells as indicated by the co- expression of CD19 and CD22.

Figure 5: The two samples images of (A) untransduced (UTD) T cells and (B) CAR T cells incubated in the presence of the controls padlock probes targeting the mRNA of two house keeping genes PSMB4 and VCP and the CAR construct. (C) The quantification of RNA transcripts targeted by the specific CART padlock probe panel indicated that 59.1% of genetically modified T cells expressed CAR molecules.

Figure 6: The panel used for the CART detection (A) Four-plex transcript detection of RNA using padlock probes and (B) the 36-plex protein detection using fluorescently-labeled monoclonal antibodies.

Figure 7 Protein staining using a 36-plex panel was performed immediately after the completion of 4-plex RNA transcription detection on human tonsil FFPE tissue sections. The four-plex RNA analysis included CAR specific RNA probe, PSMB4 and VCP positive control probes and a sequence-irrelevant negative control RNA probe. Images were counter-stained with DAPI to facilitate detection of cell nuclei. (A) Representative overlay image of human tonsil tissue section stained with CAR RNASky probe, PSMB4, VCP, negative control RNASky probes, CD3 specific antibody and DAPI. (B) Visualization of tissue section in (A), showing only positive control RNASky probes and DAPI; or (C) only CD3+ protein MICS staining for T cells.

## Claims

1. A method to detect the phenotype and the spatial location of cells provided with a chimeric antigen receptor (CAR) in a fixed biological sample by targeting the mRNA coding for the chimeric antigen receptor (CAR) comprising the steps
a. providing a plurality of oligonucleotides, each having at least one detection section and at least one amplification section and two target binding sections at the respective 5' and 3' ends
b. hybridizing the oligonucleotides with their 3' and 5' ends to the mRNA
c. ligating the 3' and 5' ends of the hybridized oligonucleotides to generate circularized molecules
d. hybridizing an amplification oligonucleotide to at least one amplification section of the circularized molecules thus enabling rolling circle amplification (RCA) of the circularized molecules
e. multiplying the circularized molecules by RCA in the fixed biological sample thereby creating DNA nanoballs comprising concatemers of the DNA nanoballs
f. hybridizing detector oligonucleotides comprising at least one fluorescent unit to at least one detection section of the DNA nanoballs, thereby obtaining the spatial location of the cells provided with the chimeric antigen receptor (CAR) in the fixed biological sample
g. providing a plurality of fluorescently-labeled antibodies capable of binding to different phenotype defining antigens, thereby obtaining the phenotypes of the cells of the fixed biological sample
h. aligning the spatial location of cells provided with the chimeric antigen receptor (CAR) and the phenotypes of cells in the fixed biological sample.

2. Method according to claim 1 **characterized in that** the mRNA coding for the chimeric antigen receptor (CAR) comprises one or more sections coding for the leader peptide, activator, hinge, skip, transmembrane, and intracellular signaling domain regions of the CAR and the oligonucleotides are provided with target binding sections at its 5' and 3' ends each capable of hybridizing to at least one of the CAR mRNA sections.

3. Method according to claim 1 or 3 **characterized in that** the plurality of fluorescently-labeled antibodies capable of binding to phenotype defining antigens are selected from the group consisting of CD11c, CD2, CD3, CD31, CD34, CD4, CD68, CD8a, Collagen1, Desmin, HLA-DR,TGF beta1, TOM22, ACTIN, CD279, CD45, CD279, CD45, CD11b, CD14, CD163, HLA-ABC, Vimentin, CD73, Ki-67, B-Catenin, Bd-2, CD-138, CD15, CD20, CD271, CD44, CD5, Fox P3, PAX-5, Podoplanin, CD16, CD274.

4. Method according to any of claims 1 to 4 **characterized in that** the expression level of the chimeric antigen receptor (CAR) within the cells in the fixed biological sample having a certain phenotype is quantified by detecting the amount of detector oligonucleotides bound to the detection sections of the DNA nanoballs.

5. Method according to any of claims 1 to 4 **characterized in that** the cells provided with a chimeric antigen receptor (CAR) have the phenotype of a T-cell.
